Europäisches Patentamt

European Patent Office

Office européen des brevets

(19)

(11) Publication number: **0 127 328**
A2

# (12) EUROPEAN PATENT APPLICATION

(21) Application number: 84302866.3

(22) Date of filing: 27.04.84

(51) Int. Cl.³: **C 12 N 15/00**
C 12 P 21/02, C 12 N 1/20
//(C12N1/20, C12R1/07, 1/185,
1/38, 1/465)

(30) Priority: 28.04.83 US 489327

(43) Date of publication of application:
05.12.84 Bulletin 84/49

(84) Designated Contracting States:
AT BE CH DE FR GB IT LI LU NL SE

(71) Applicant: GENEX CORPORATION
6110 Executive Boulevard
Rockville Maryland 20852(US)

(72) Inventor: Saunders, Charles W.
12505 Village Square Terrace
Rockville Maryland 20852(US)

(72) Inventor: Fahnestock, Stephen R.
2904 Gold Mine Road
Brookeville Maryland 20833(US)

(72) Inventor: Guyer, Mark S.
5710 Roosevelt Street
Bethesda, MD 20817(US)

(72) Inventor: Banner, Carl
4601 Stoneleigh Ct.
Rockville, MD 20852(US)

(74) Representative: Holmes, Michael John et al,
Frank B. Dehn & Co. European Patent Attorneys Imperial
House 15-19 Kingsway
London, WC2B 6UZ,(GB)

(54) The use of chromosomal integration to stabilize heterologous genes.

(57) Disclosed is a method of expressing a heterologous gene in a prokaryotic microorganism wherein the gene is one which cannot be maintained stably in the microorganism when carried on a plasmid vector, which comprises integrating the gene into the chromosome of the microorganism. Also disclosed are techniques for production of polypeptides for which the genes code, and micro-organisms containing such genes.

THE USE OF CHROMOSOMAL INTEGRATION TO STABILIZE
HETEROLOGOUS GENES

This invention relates to a method of stabilizing the inheritance of heterologous genes or gene fusions which are unstable in bacteria when they are borne on plasmid vectors.

## Background of the Invention

One of the important uses to which the techniques of in vitro DNA recombination have been put is the construction of microbial strains, and other cellular systems, which make high levels of one or a few proteins of particular interest. Such proteins may have either commercial or research value and may be of homologous (encoded by a gene obtained from the host organism) or of heterologous (encoded by a gene from an organism other than the host) origin.

The synthesis of such a genetically engineered protein (or, equivalently, the expression of a genetically engineered gene) requires initially that the DNA sequence encoding the protein be "transcribed" (copied into a messenger RNA). Transcription is initiated at a specific signal or site in the DNA known as the promoter. Next, the messenger RNA sequence must be "translated" into the primary polypeptide structure of the protein. This process begins when a ribosome binds to a specific signal or site in the messenger RNA known as the translation initiation site. In many cases, the ability of a gene to be expressed in a heterologous host requires that its natural promoter and/or translation initiation site be replaced (using recombinant DNA technology) by signals which can be

recognized by the expression systems of the new host. The use of such gene fusions is required in cases in which the new host is unable to recognize the expression signals naturally associated with the gene in its host of origin. Gene fusions can also be used to alter the properties of gene products of interest in other ways, for instance to affect or optimize the ability of a protein to be secreted or to alter the biochemical or biophysical properties of a protein to make purification easier. For the purpose of this disclosure, the behavior of a heterologous gene with natural expression signals or one which has been altered in any one of a variety of ways (as described above) can be considered to be equivalent. Thus the term "heterologous gene" will be used to refer to both situations.

A problem which is often encountered by those working with recombinant DNA, particularly in the development of heterologous gene expression systems, is the inability to isolate or maintain bacterial strains carrying certain chimeric plasmids. This problem can be manifested as the failure to find transformants carrying the chimeric plasmid (T. Imanaka et al., J. Bacteriol., 147: 776-786 (1981); D. Stassi and S.A. Lacks, Gene 18: 319-328 (1982)). In other cases, some transformants are recovered but these rapidly lose the chimeric plasmid (C. Donnelly and A.L. Sonenshein, in Molecular Cloning and Gene Regulation in Bacilli, Ed. A.T. Ganesan et al., Academic Press, New York, 1982, pp. 63-72). In yet other cases, some stable transformants are recovered, but these contain a chimeric plasmid that has suffered rearrangement (C. Donnelly and A.L. Sonenshein, supra; G. Grandi et al.; Plasmid 6: 99-111 (1981); J. Kreft et al., in A.T. Ganesan et al., supra, pp. 145-157; M. Uhlen et al., Plasmid, 5:161-169 (1981)).

A number of hypotheses have been advanced to account for the unstable nature of such chimeric plasmids. These include (1) the production of large amounts of a gene product which is inhibitory or even lethal to the host cell, or (2) the saturation of an essential cellular component by the cloned gene product or the expression system (N.E. Harding et al., J. Bacteriol., 152:983-993 (1982); P.W. Rice and J.E. Dahlberg, J. Bacteriol., 152: 1196-1210; T. Imanaka et al., supra; D. Stassi and S.A. Lacks; supra). Both of these models propose that the problem is the excessive expression of the cloned gene, expression above a threshhold level such that the ability of the host cell to grow is impaired. Cells in which such excessive expression occurs either die or grow slowly and are supplanted in the population by other cells which either have lost the plasmid or in which the DNA of the plasmid has rearranged so that expression is reduced to an acceptable level.

A third possiblity is that expression of a particular plasmid-borne gene above a certain level can disrupt a plasmid function or functions which are required for establishing or maintaining the plasmid (D. Steuber and H. Bujard, EMBO Journal, 1:1399-1404 (1982)). Excessive expression in this case would be at a level which is high enough to prevent either the establishment of the plasmid or the normal distribution of the plasmid to daughter cells upon cell division. This would result in the failure of cells which had lost the plasmid to grow under conditions for which the presence of the plasmid is required (e.g., in the presence of antibiotics).

Although it is not currently known which one of these or other, theories provides the actual explanation for the instability of a chimeric plasmid in a given situation, a common underlying principle is that excessive gene expression prevents the recovery of stable transformants containing the chimeric plasmid. An object of this invention thus is to develop a technique for introducing and stably maintaining genes which, for whatever reason, are unstable because of excessive expression when carried on plasmid vectors.

According to the invention there is provided a method of expressing a heterologous gene in a prokaryotic microorganism wherein the gene is one which cannot be maintained stably in the microorganism when carried on a plasmid vector, which comprises integrating the gene into the chromosome of the microorganism.

The invention also provides a method as described above in which the prokaryotic microorganism, the chromosome of which has said heterologous gene integrated therein, is cultivated under polypeptide producing conditions to produce the polypeptide for which said heterologous gene codes.

There is also provided a prokaryotic microorganism the chromosome of which has been modified by integration therein of a heterologous gene as described above.

The integration of a heterologous gene into the chromosome of a microorganism has two effects. It generates a strain in which a single copy of the DNA sequence is present per chromosomal equivalent. This will reduce the gene dosage and if the consequent level of the gene product is below the threshhold at which cellular growth is impaired, the strain will not be unstable.

Secondly, an integrated heterologous gene is no longer dependent on a plasmid maintenance system for its inheritance. Thus instability due to disruption of an essential plasmid function will not be a problem. Whether or not instability which has been observed is due to the effects described, the method of this invention has been shown to stabilize such otherwise unstable genes.

There are several techniques which can be used to effect the integration of a heterologous gene or gene fusion into the chromosome of a microorganism. In one embodiment, the gene or gene fusion of interest is cloned into a plasmid which is capable of integrating, by homologous recombination, into the chromosome of a microorganism. A plasmid of this type is known as a chromosomal integration vector and is defined as a vector which (a) contains a replicon (the genetic functions which are required for it to be maintained as a plasmid) which functions in one host microorganism (e.g., E.coli) but not in another (e.g. B.subtilis); (b) contains genetic markers which can be used for selection in each host; (c) contains a DNA sequence (or sequences) which is homologous to the chromosome of the second microorganism (in the example given, B.subtilis). Because of the presence of a plasmid replicon which functions in one microorganism, the chromsomal integration vector can be maintained and manipulated easily in that host. This construction allows all of the necessary cloning and genetic engineering procedures to be done readily. Because of the presence of genetic markers which can function in both hosts, the presence of the plasmid can be selected or detected in each microorganism. Finally, the presence of a DNA sequence homologous to a region of the chromosome of the second microorganism allows the vector, upon transformation into that host, to be integrated into the chromosome of that host by virtue of recombination between the homologous sequences on the vector and the chromosome. There is little or no specificity in terms of what regions of the host chromosome can serve as regions of homology; of the many restriction fragments which are generated upon digestion of the chromosome of a microorganism, the vast majority will adequately serve to provide the required homology. There may, however, be differences in terms of the frequencies with which individual fragments promote integration (see Table 2). These differences depend on the differences in length, DNA base composition and sequence, and chromosomal location. In situations in which a high frequency of integration is desired, the use of a particular empirically-identified fragment may be preferable. The vector cannot be maintained as an extrachromosomal element in the second host because it does not include a replicon which is functional in that organism.

A chromosomal integration vector can be used as a cloning vector to insert a DNA sequence, such as a restriction fragment containing a gene of interest. When such a gene is cloned and the resulting plasmid is used to transform the second microorganism, the gene of interest will be integrated into its chromosome along with the rest of the chromosomal integation vector. A variation of this approach, in which the gene of interest is flanked by regions of homology which normally are located close together on the host chromosome, may result in substantially higher transformation frequencies.

Chromosomal integration vectors have been reported in the literature, but discussions of their usefulness heretofore have focused on their use in genetic analyses of microorganisms such as Bacillus subtilis and E.coli (K. Yamaguchi and J. Tomizawa, Mol. Gen. Genet. 178: 525-533 (1980); T. Date, J. Bacteriol. 154: 76-83 (1983); F.E. Wilson et al., J. Bacteriol. 148: 624-628 (1981); W.G. Haldenwang, et al., J. Bacteriol., 142: 390-98 (1980); E. Ferrari and J.A. Hoch, Mol. Gen. Genet. 189: 321-325 (1983)). Chromosomal integration vectors have not previously been used as a means for achieving the stable maintenance and expression of heterologous genes in microorganisms.

In a second embodiment, the gene of interest can be cloned into a vector which, as isolated from nature, has the capacity to integrate into the chromosome of a host microorganism, either by generalized or specialized recombination. Generalized recombination is that which requires homology between the vector and the host; specialized recombination does not require homology. Such a vector may be a temperate bacteriophage (F. Kawamura et al., Gene, 5: 87-91 (1979); Y. Yoneda et al., Biochem. Biophys. Res. Comm., 91: 1556-1564 (1979)) or a plasmid (J. Hofmeister et al., Mol. Gen. Genet., 189: 58-63 (1983); Y. Nishimura et al., J. Mol. Biol., 55: 441-456 (1971)) or a transposon (N.J. Grinter, Gene; 21: 133-143 (1983)).

Introduction into a microorganism of any of the above vectors which are capable of integrating into the chromosome of that microorganism generates a strain in which the DNA sequence of the vector itself becomes a chromosomal sequence. Subsequent transformation of that strain with a distinguishable vector which is partially homologous to the first vector and which contains the gene of interest will result in integration of the second vector (by recombination with the first), even if the second vector contains no DNA sequence derived from the chromosome of the original microorganism (E. Ferrari and J.A. Hoch, supra). Thus, secondary transformation of a strain already carrying an integrated vector sequence is a third means of achieving the chromosomal integration of a gene of interest.

Each use of one of the types of vectors described results in the introduction of one copy of the heterologous gene into the chromosome of the host microorganism. If the host can tolerate more than a single copy without the inheritance becoming unstable, multiple copies can be inserted (one at a time) by repeated use of one or more of the types of vectors described.

Reports in the literature regarding the types of vectors described in these alternate embodiments of the invention have not demonstrated that such vectors can be used as a way of stabilizing otherwise unstable heterologous genes in prokaryotic organisms. The method of this invention provides a novel way to stabilize in various bacteria a variety of genes the expression of which interferes with the stable inheritance of chimeric plasmids which contain them. It thus allows high levels of production of proteins derived from prokaryotic and eukaryotic sources to be achieved. Such proteins may be intracellular or extracellular in location and may be native or modified (for purification or other purposes) in configuration. The host microorganism may be either a Gram-positive or a Gram-negative organism. Gram-positive microorganisms include those selected from the genus Bacillus, the genus Streptomyces and the Coryneform group of bacteria. Gram-negative microorganisms include those selected from the genus Escherichia and the genus Pseudomonas. In a preferred embodiment of this invention the host microorganism is B.subtilis.

The following examples are intended to illustrate how to carry out the method of the present invention and are not to be construed as limiting.

Example I

Construction of a Chromosomal Integration Vector

For the DNA manipulations described in this and the following examples, the restriction endonucleases and other enzymes used were purchased from New England BioLabs, Inc., Bethesda Research Laboratories, Inc. or Boehringer Mannheim GmbH, Biochemica and were used as recommended by the manufacturer unless indicated otherwise.

A. Construction of Plasmid pGX345

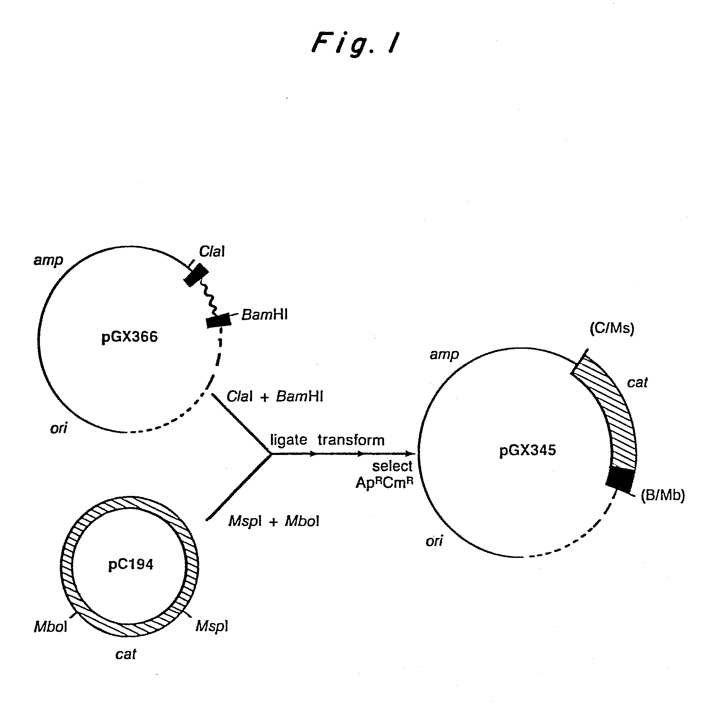

Plasmid pGX366 (Figure 1) was digested with ClaI and BamHI and used to clone a 1 kilobase (kb) DNA fragment from pC194 (T.J. Gryczan et al. J. Bacteriol., 134: 318-329 (19-78); S. Horinuchi and B. Weisblum, J. Bacteriol., 150: 815-825 (1982)). This fragment contains cat, a gene which specifies resistance to chloramphenicol in B.subtilis; the fragment lacks a replicon which is functional in that organism.  It was generated by digesting pC194 with MspI (which generates the same 5' overhang as ClaI) and with MboI (which generates the same 5' overhang as BamHI). After ligation, E.coli cells were transformed as described (M.S.  Guyer, "The γδ Sequence of F is an Insertion Sequence," J. Mol. Biol., 126: 347-365 (1978)). Transformants were selected on L agar (1% tryptone (Difco Laboratories, Inc., Detroit), 0.5% yeast extract (Difco), 1% NaCl, 1.5% agar (Difco)) containing 30 μg/ml of ampicillin and 10 ug/ml of chloramphenicol.  The resulting cat-containing plasmid (Figure 1) was designated pGX345.

In Figure 1 and subsequent figures, the following legend applies:

_____ indicates DNA originally derived from pBR322 (specifically in pGX366, the DNA sequence from the PvuII site to the HindIII site),

---------- indicates DNA from the galK region of E.coli (K. McKenney et al. in Gene Amplification and Analysis, Vol. II, Ed. J. Chirikjian et al., Elsevier, North Holland (1981)),

— — — indicates DNA from the 4S transcriptional termination region of coliphage lambda (K. McKenney et al., supra),

▨▨▨▨ indicates a synthetic multi-restriction site sequence from pUC9 (J. Messing and J. Vieira, Gene 19: 269-276 (1982),

∿∿∿ indicates DNA from the blaZ region of S.aureus plasmid pI258 (R.P. Novick et al., Plasmid, 2:109-129 (1979)

▨▨▨ indicates DNA from pC194,

▨▨▨ indicates DNA from the amyBA2 region of B.amyloliquifaciens (see example IV)

********** indicates DNA from the <u>pra</u> region of <u>S.aureus</u> (see example III),

<u>⌐___¬</u> indicates DNA from the chomosome of

or <u>ᴠᴠᴠᴠ</u> <u>B.subtilis</u>,

(C/Ms) indicates a hybrid <u>ClaI</u>/<u>MspI</u> site, (B/Mb) indicates a hybrid <u>BamHI</u>/MboI site, and (N/P) indicates a hybrid <u>NruI</u>/<u>PvuII</u> site. The figures are not drawn to scale.


B. Construction of Plasmids pGX281 and pGX282

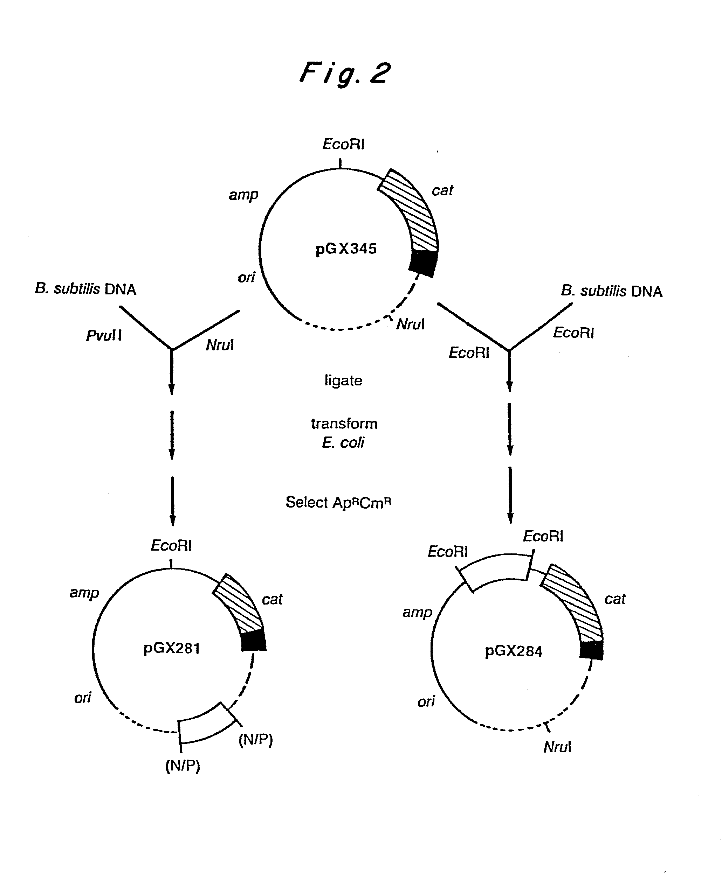

Plasmid pGX345 was digested with <u>NruI</u>, a restriction enzyme which generates blunt ends. DNA from <u>Bacillus subtilis</u> strain BR151 (P.S. Lovett, et al., <u>J. Bacteriol.</u>, 127: 817-828 (1976)) was digested with <u>PvuII</u>, an enzyme which also generates blunt ends. The opened pGX345 and the <u>B.subtilis</u> DNA were ligated, then transformants were isolated as described in the isolation of pGX345. From this experiment, strains containing plasmids designated as pGX281 (which contains a 1.1 kb insert of <u>B.subtilis</u> DNA) and pGX282 (which contains an 8 kb insert of <u>B.subtilis</u> DNA) were isolated (see Figure 2, Table 1). <u>B.subtilis</u> strain BR151 was transformed with pGX281 and Cm$^R$ transformants, including strain GX2499, were isolated. <u>B.subtilis</u> strain BR151 was made competent and transformed by a modification of the method of C. Anagnostopoulos and J. Spizizen (<u>J. Bacteriol.</u>, 81: 741-746 (1961)). To prepare competent cells, <u>B.subtilis</u> strain BR151 was grown overnight at 37°C on L agar or tryptose blood agar base (Difco). Cells were resuspended in 10 ml of SPI medium (supplemented with 50 µg/ml each of lysine, tryptophan, and methionine) to give a reading of 50-70 on a Klett-Summerson colorimeter equipped with a green filter (Klett Mfg. Co., New York). SPI medium consists of 1.4% $K_2HPO_4$, 0.6% $KH_2PO_4$, 0.2% $(NH_4)_2SO_4$, 0.1% sodium citrate·2 $H_2O$, 0.5% glucose, 0.1% yeast extract (Difco), 0.02% Bacto-Casamino acids (Difco), and 0.02% $MgSO_4·7H_2O$.

The cultures were incubated at 37°C on a rotary shaker (200-250 rpm) for 3 to 4 1/2 hr. until logarithmic growth began to cease and the cells entered early stationary phase. The cells were then diluted 10 fold into the same medium supplemented with 0.5 mM $CaCl_2$. Incubation was continued for 90 min. longer. The cells were then centrifuged for 5 min. at room temperature and resuspended in 1/10 volume of spent medium. One ml aliquots of the cell suspensions were frozen in liquid nitrogen and stored at -80°C for use.

For transformation, the frozen competent cells were thawed quickly at 37°C and diluted with an equal volume of SPII medium supplemented as above with amino acids. SPII is the same as SPI except that the concentration of $MgSO_4$ is increased to 0.04% and 2mM ethyleneglycol-bis-(β-aminoethyl ether)-N,N,N',N'-tetraacetic acid (EGTA) was added. 0.5 ml of cells were mixed with 0.1 to 5 ug of DNA in 13x100 mm glass tubes. The cell suspensions were incubated at 37°C in a reciprocal shaking water bath (Fisher Scientific Co., setting 6) for 30 min. 0.1 ml samples were added to 2.5 ml of molten 0.7% Bacto-agar (Difco) at 48° and poured onto L agar plates. The plates were incubated at 37°C for one hour; then they were overlayed with 2.5 ml of molten 0.7% agar containing antibiotics appropriate for selection of desired transformants (final concentration in the plates of 10 ug/ml of chloramphenicol or 5 µg/ml of Kanamycin). The plates were incubated overnight at 37°C. Cellular DNA from GX2499 was analyzed by gel transfer hybridization (G.M. Wahl et al., Proc. Natl. Acad. Sci. 76:3683-3687 (1979)), using nick-translated (P.W.J. Rigby et al., J. Mol. Biol., 113:237-251 (1977)), linearized pGX2413 (see below) as a probe. After digestion of the cellular DNA with any of several restriction enzymes, or combinations of enzymes, the hybridization patterns of the DNA were consistent with the patterns predicted if a single copy of the entire vector had inserted into the site of homology on the B.subtilis chromosome (Table 1). The patterns were inconsistent with those predicted in either of two other cases, if there were free plasmid in the cells or if multiple copies (either tandem or head to head) of the plasmid had integrated into the chromosome.

## Table 1

### Summary of Results of Southern Blot Experiments with GX2499

| Enzyme used to digest chromosomal DNA | Size (in kb) of fragments which Hybridized | Size (in kb) of fragments predicted to hybridize if the transforming plasmid were established as: | | |
|---|---|---|---|---|
| | | single chromosomal copy | multiple tandem chromosomal copies | free plasmid |
| PvuII | 7 | 6.8 | 13 (18,...) | 5.7 |
| BglII | 10 | 10 | 15 (21,...) | 5.7 |
| BglI + PvuII | 3.4 3.5 | 3.4 3.5 | 3.4 3.5 5.7 | 3.4 3.5 5.7 |

## C.   Construction of Plasmid pGX284

Plasmid pGX345 was digested with EcoRI and used to clone EcoRI fragments of B.subtilis DNA (obtained from a derivative of strain BR151). Transformants were isolated as described above and integration vectors designated pGX284-pGX289, inclusive, were isolated. The B.subtilis DNA insert in plasmids of this group ranges from <0.6 to 5 kb in size (Table 1; note that some vectors have acquired two fragments of B.subtilis DNA). The ability of different integration vectors to transform B.subtilis varies over a hundred-fold range (Table 2) and is presumably dependent upon the nature of the particular insert carried by each plasmid.

## Table 2

### Transformation of BR151 with Chromosomal Integration Vectors

| DNA | Insertion size (kb) | CmR Transformants[a] |
|---|---|---|
| – | – | <1 |
| pGX345 | – | <1 |
| pGX281 | 1 | 5 |
| pGX282 | 8 | 145 |
| pGX284 | 1, 2 | 39 |
| pGX285 | 1, 2 | 220 |
| pGX286 | 2.5 | 600 |
| pGX288 | 3, 3.5 | 24 |
| pGX289 | 5 | 475 |
| pGX2413 | 1 | 2 |
| pGX2414 | 1 | 3 |

[a]For the experiment described in Table 2, donor DNA was mixed with competent cells at a concentration of 1 µg/ml. The number of CmR transformants obtained by plating 0.1 ml of the transformation mixture is listed.

## Example II

### Stabilization of blaZ

blaZ is a gene which is found in the S.aureus plasmid pI258 (R.P. Novick et al., supra) and which encodes a β-lactamase. K. Timmis et al. (Proc. Nat. Acad. Sci., U.S.A., 72: 2242-2246 (1975)) cloned a 7 kilobase (kb) EcoRI fragment from pI258 into pSC101, creating pSC122.

Next, repeating the experiment of J. R. McLaughlin et al., (J. Biol. Chem., 256: 11273-11282 (1981)), the blaZ-containing EcoRI fragment was recloned from pSC122 into the higher copy number replicon pMB9 (F. Bolivar et al., Gene, 2:75-93 (1977)), generating pGX300. To reduce the amount of pI258-derived DNA present, pGX300 was digested with HindIII and EcoRI and self-ligated. Among the Ap$^R$ transformants recovered, two strains containing smaller plasmids were isolated; these plasmids have been designated pGX311 and pGX310.

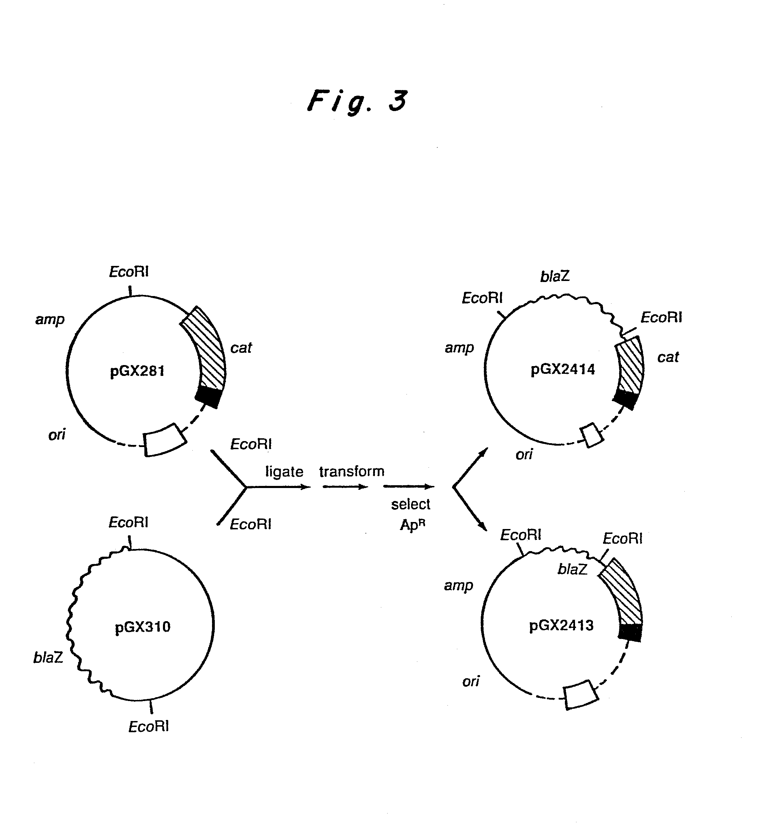

pGX281 was digested with EcoRI and used as a vector to clone an EcoRI fragment, which contained blaZ, from pGX310 (Figure 3). Transformants were isolated as described for pGX345. Among the Ap$^R$Cm$^R$ isolates obtained were GX2489, a strain containing a plasmid designated pGX2413, and GX2490, a strain containing a plasmid designated pGX2414. These two plasmids differ only in the orientation with which the blaZ fragment is inserted into pGX281 (Figure 3). pGX2413 and pGX2414 transform B.subtilis to Cm$^R$ at the same frequency as does the pGX281 parent (Table 2). The pGX2413 and pGX2414 transformants produce β-lactamase (β-lactamase production was assayed using Cefinase (the Beckton Dickinson and Co.) discs as recommended by the supplier (BBL Microbiology Systems, Cockeysville, MD)). The ability to produce β-lactamase is stable during growth in the absence of selection for retention of cat (Table 3).

In contrast to the behavior of the integrated blaZ, several attempts to clone a gene fragment containing blaZ in B.subtilis using multicopy plasmid vectors were unsuccessful. For example, in an attempt to establish blaZ in B.subtilis, potential shuttle vectors were constructed from pGX311. HindIII-digested pC194 was inserted at the HindIII site of pGX311, generating a plasmid designated pGX314; similarly, BamHI-digested pUB110 (T.J. Gryczan et al., supra) was inserted at the BamHI site of pGX311, generating a plasmid designated pGX312. pGX312 and pGX314 transformed competent BR151 cells to $Km^R$ and $Cm^R$ respectively at very low efficiency. In all cases tested, the few transformants obtained harbored plasmids that differed significantly in size and restriction pattern from the parental plasmids. In particular, blaZ did not appear to be intact in any of these transformants.

## Table 3

### Stability of B.subtilis Strains Carrying Integrated Plasmids

| Strain | Integrated Plasmid | Stability[a] | | | |
| | | Plate Transfers | | Liquid Transfers | |
| | | $Cm^R$/ Total | $Bla^+$/ Total | $Cm^R$/ Total | $Bla^+$/ Total |
|---|---|---|---|---|---|
| GX2495 | pGX281 | 33/32 | 0/9 | 7/7 | 0/1 |
| GX2494 | pGX2413 | 11/11 | 10/10 | 17/17 | 6/6 |
| GX2493 | pGX2414 | 39/39 | 6/6 | 13/13 | 4/4 |

[a]To test the stability of strains carrying integrated vectors, several strains were sub-cultured by patching daily onto drug-free L agar plates for 6-10 days. The samples were streaked out onto drug-free L agar. Single colonies then were picked and scored for chloramphenicol resistance ($Cm^R$) and β-lactamase production ($Bla^+$). As another test, cultures were grown in drug-free liquid medium for 30 generations. Single colonies were isolated on L agar and scored for chloramphenicol resistance and β-lactamase production.

## Example III
## Stabilization of pra

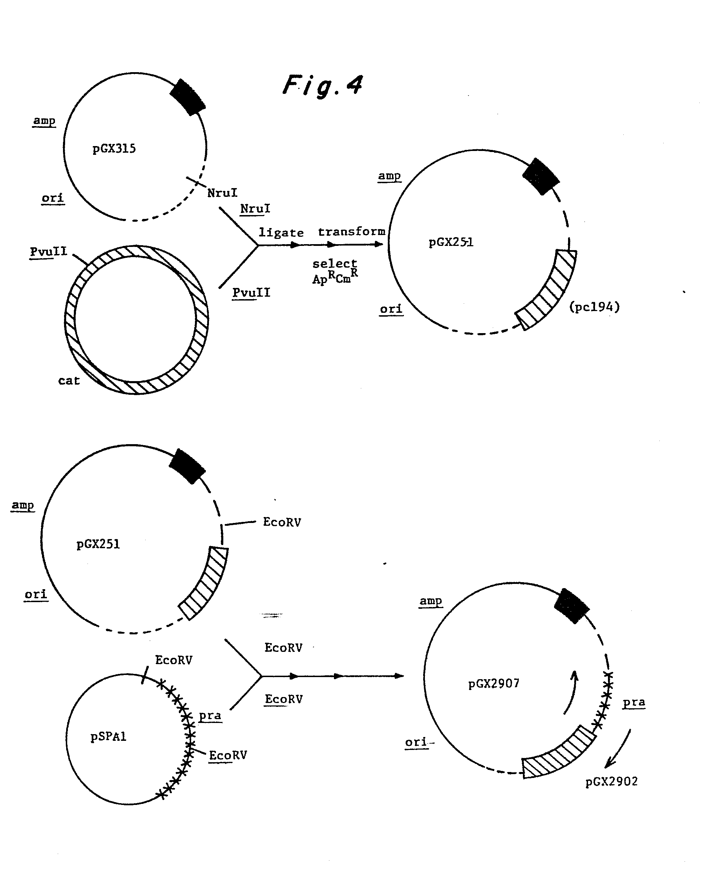

The gene pra, isolated from a strain S.aureus, encodes protein A. The gene pra was subcloned from pSPAl (S. Lofdahl et al., Proc. Nat. Acad. Sci. U.S.A., 80: 697-701 (1983)) into the shuttle vector pGX251 (pGX251 was made (Figure 4) by ligating PvuII-digested pC194 with NruI-digested pGX315, transforming E.coli and selecting Ap$^R$Cm$^R$ transformants. By definition, a shuttle vector is a vector capable of replicating in two hosts; pGX251 can replicate in both E.coli and B.subtilis). pGX251 was digested with EcoRV and used to clone the EcoRV fragment of pSPAl which contains pra (Figure 4). Transformants were isolated as described for pGX345. Among the Ap$^R$Cm$^R$ transformants of E.coli isolated were GX3311, a strain containing the plasmid designated pGX2901, and GX3312, a strain containing a plasmid designated pGX2902. The two plasmids differ only in the orientation with which the pra fragment is inserted into pGX251 (Figure 4).

No transformants were obtained from attempts to transform B.subtilis BR151 with pGX2901. A few Cm$^R$ colonies were obtained from transformation with pGX2902. These, however, were very unstable as shown for one such isolate, GX3308 (Table 4). Thus, pra is another gene which cannot be stably maintained on at least one plasmid vector in B.subtilis.

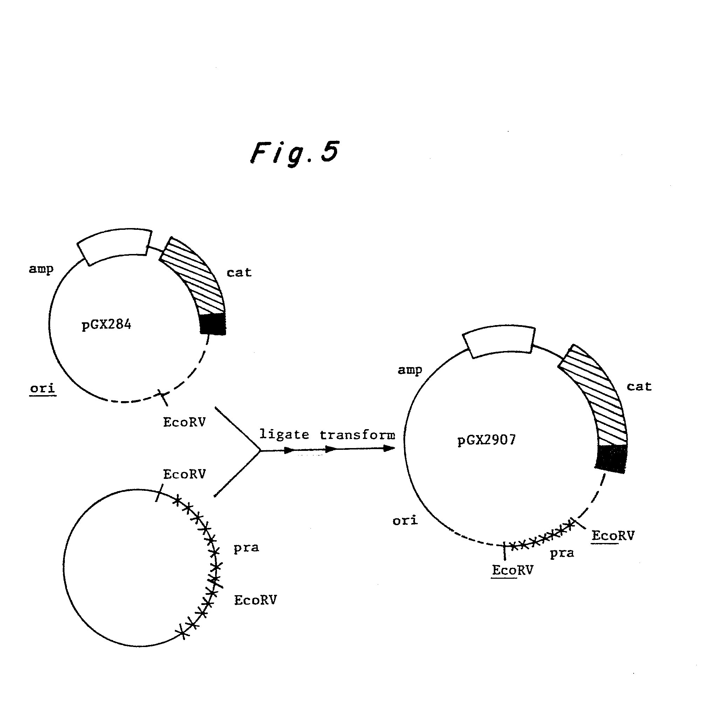

To make pra-containing integration vectors for comparison with these results, pGX284 was digested with EcoRV and used to clone the pra-containing EcoRV fragment of pSPAl (Figure 5). Among the Ap$^R$ transformants of E.coli obtained, one strain, GX3320, contained plasmid pGX2907 which carried one copy of the pra EcoRV fragment and another strain, GX3320-2, carried pGX2907-2, which carried two copies of the pra EcoRV fragment. Transformed strain GX3320 has been deposited with American Type Culture Collection, Rockville, MD. and has been designated ATCC No. 39344.

Both pGX2907 and pGX2907-2 transformed B.subtilis and gave rise to Cm$^R$ transformants which were much more stable than GX3308 (Table 4). Although the results described in Table 4 suggest some variability in stability from strain to strain and from experiment to experiment with strains which contain chromosomally integrated pra, such strains are clearly much more stable than strains in which pra is carried on a plasmid.

18

## Table 4
## Stability of B.subtilis Strains Carrying pra

| Strain | Number of Generations[d] | %Cm$^R$Pra$^{+e}$ | |
|---|---|---|---|
| | | Expt 1 | Expt 2 |
| GX3302-2[a] | 5 | 100 | 100 |
| | 15 | 70 | 64 |
| | 25 | 5 | 67 |
| | 35 | 10 | 60 |
| GX3305[b] | 5 | 100 | -- |
| | 15 | 95 | -- |
| | 25 | 100 | -- |
| | 35 | 80 | -- |
| GX3305-2[c] | 5 | 100 | -- |
| | 15 | 100 | -- |
| | 25 | 95 | -- |
| | 35 | 85 | -- |
| GX3308 | 5 | <5 | -- |
| | 15 | <5 | -- |

[a]GX3302-2 is BR151 transformed with pGX2907-2

[b]GX3305 is B.subtilis 1S53 (spoOAΔ677, obtained from the Bacillus Genetic Stock Center) transformed with pGX2907 (ATCC No. 39345).

[c]GX3305-2 is BR151 transformed with pGX2907-2.

[d]Cells were grown for the indicated number of generations in AM3 (antibiotic medium number 3 (Difco)).

[e]Protein A was assayed as described by Lofdahl et al. (supra).

## Example IV
## Stabilization of amyBA2

Chromosomal DNA of B.amyloliquifaciens ATCC 23844 was partially digested with MboI (using reaction conditions

which gave an average fragment size of 4 kb; the reaction was terminated by addition of an equal volume of phenol: chloroform (1:1)). The digest was ligated (at a molar ratio of 1:1 and a total DNA concentration of 300 µg/ml) to the BamHI-digested vector pBD64 (T.J. Gryczan et al., J. Bacteriol., 141: 246-253 (1980)). The ligated DNA was used to transform B.subtilis strain IS240 carrying pUB110. Strain IS240 was used because it is a low amylase producer, but in general any Bacillus subtilis 168 derivative carrying pUB110 which produces α-amylase at wild type levels, such as BR151, could be used for this cloning.

Clones producing α-amylase (Amy$^+$) were identified by the production of zones of clearing on precipitated starch agar plates (tryptose blood agar base (Difco) containing 1% soluble starch (Sigma) stored at 4°C for at least three weeks) with 10 ug/ml chloramphenicol. Plasmid DNA was extracted from Amy$^+$ clones by the method of Birnboim and Doly (Nuc. Acids. Res., 7: 1513-1523 (1979)) and used to transform BR151. One Amy$^+$Cm$^R$ transformant was designated GX2508 and contained a plasmid designated pGX2508. The gene on pGX2508 encoding the α-amylase activity was designated amyBA2 to distinguish it from other α-amylase genes.

To reduce the amount of B.amyloliquifaciens DNA in the plasmid, a PvuII to BamHI amyBA2-containing fragment of pGX2508 was sub-cloned into pBD64, generating plasmid pGX2509. Experiments have shown that a strain containing this plasmid, GX2509, which is BR151 carrying pGX2509, is an unstable producer of α-amylase. After incubation for 48 hours in AM3 medium in the absence of any selection for retention of the plasmid, only about 0.1-0.2% of the cells retained the Amy$^+$ phenotype (i.e., only 5 of ca. 3000 examined colonies were Amy$^+$).

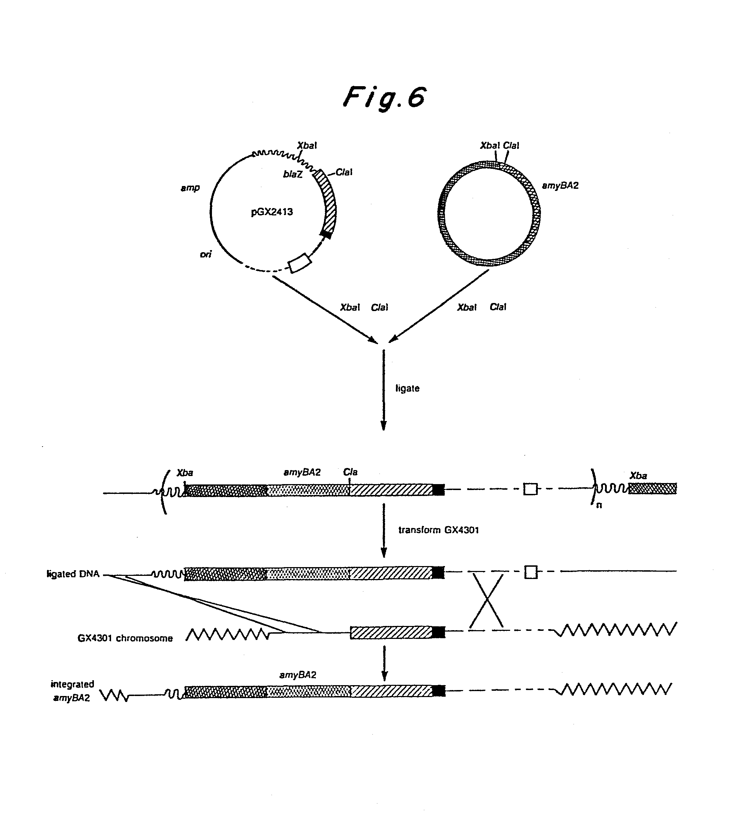

For comparison, amyBA2 was integrated into the chromosome of B.subtilis by a modification of the methods of Ferrari and Hoch (supra) and Kawamura et al. (supra). Plasmids pGX2413 and pGX2509 were each digested with both ClaI and XbaI and ligated (at a molar ratio of 1:1 and a total DNA concentration of 100 ug/ml; under these conditions the ligation products tend to be long, concatemeric linear molecules rather than circular molecules). The ligated DNA was used to transform GX4103, which is BR151 carrying an integrated copy of pGX281). Amy$^+$ clones were obtained by congression with a Met$^+$ marker (C.P. Moran, Jr. et al., J. Bacteriol., 142: 331-334 (1980)); these transformants were presumably derived from an amy-containing fragment of pGX2509 flanked by pGX2413 sequences, followed by recombination between the homologous regions of pGX2413 and the integrated pGX281 sequence (Figure 6). One such Amy$^+$ clone has been designated GX2510. After incubation of GX2510 for 48 hours in AM3 medium in the absence of selection, at least 90% (13/13) of the cells retained the Amy$^+$ phentoype, in contrast to the unstable behavior of GX2509.

Claims

1. A method of expressing a heterologous gene in a prokaryotic microorganism wherein the gene is one which cannot be maintained stably in the microorganism when carried on a plasmid vector, which comprises integrating the gene into the chromosome of the microorganism.

2. A method in accordance with claim 1 wherein

(a) the heterologous gene is inserted into a chromosomal integration vector which contains:

(i) a phenotypic marker which can be expressed and selected for in a first microorganism;

(ii) a phenotypic marker which can be expressed and selected for in a second microorganism;

(iii) a functional replicon which allows the vector to be maintained in the first microorganism, and

(iv) a DNA sequence which is homologous to a region of the chromosome of the second microorganism; and

(b) the resulting chimeric plasmid, comprised of the chromosomal integration vector and the gene of interest, is used to transform the second microorganism such that the DNA sequence of the chimera which is homologous to a DNA sequence of the chromosome of the second microorganism recombines with that chromsomal sequence, and the gene inserted into the integration vector is integrated into the chromosome.

3. A method in accordance with claim 1 wherein

(a) the heterologous gene is inserted into a vector which, as isolated from nature, has the ability to integrate into the chromosome of the prokaryotic microorganism, and

(b) the resulting chimeric DNA transforms or infects the prokaryotic microorganism.

4. A method in accordance with claim 3 wherein the vector is a temperate bacteriophage.

5. A method in accordance with claim 3 wherein the vector is a plasmid.

22        0127328

6.   A method in accordance with claim 1 wherein the vector is a transposon.

7.   A method in accordance with claim 1 wherein

(a) the prokaryotic microorganism is transformed with a first vector capable of integrating into the chromosome of that microorganism;

(b) a heterologous gene is inserted into a second vector which is partially homologous to the first vector; and

(c) the strain generated by the transformation of the microorganism of step (a) is transformed with the chimeric DNA sequence of step (b) such that the heterologous gene is integrated into the chromosome of the microorganism.

8.   A method in accordance with claim 1, 2, 3, 4, 5, 6, or 7 wherein the prokaryotic microorganism is a Gram-positive microorganism.

9.   A method in accordance with claim 8 wherein the microorganism is the genus <u>Bacillus</u>.

10.   A method in accordance with claim 9 wherein the microorganism is of the species <u>subtilis.</u>

11.   A method in accordance with claim 8, wherein the microorganism is of the genus <u>Streptomyces.</u>

12.   A method in accordance with claim 8, wherein the microorganism is of the Coryneform group.

13.   A method in accordance with claim 1, 2, 3, 4, 5, 6 or 7 wherein the prokaryotic microorganism is a Gram-negative microorganism.

14.   A method in accordance with claim 13 wherein the microorganism is of the genus <u>Escherichia.</u>

15.   A method in accordance with claim 14 wherein the microorganism is of the species <u>coli</u>.

16.   A method in accordance with claim 13 wherein the microorganism is of the genus <u>Pseudomonas.</u>

0127328

17. A method for preparing a prokaryotic microbial strain capable of expressing a polypeptide which is normally exogenous to sàid organism which comprises the steps of:

(a) transforming prokaryotic cells with a chromosomal integration vector which contains

(i) a DNA sequence coding for said polypeptide;

(ii) a phenotypic marker which can be expressed in said prokaryotic cells;

(iii) a DNA sequence which is homologous to a region of the chromosome of said prokaryotic cells which region is capable of directing the integration of the vector into the chromosome; and

(b) isolating transformants having the trait specified by the phenotypic marker.

18. A method for producing a polypeptide which comprises cultivating, under polypeptide-producing conditions a prokaryotic microbial strain prepared in accordance with the method of claim 17 on a nutrient medium containing essential assimilable nutrients.

19. A method as claimed in claim 1 in which the prokaryotic microorganism, the chromosome of which has said heterologous gene integrated therein, is cultivated under polypeptide producing conditions to produce the polypeptide for which said heterologous gene codes.

20. A prokaryotic microorganism the chromosome of which has been modified by integration therein of a heterologous gene, wherein the gene is one which cannot be maintained stably in the microorganism when carried on a plasmid vector.

*Fig. 1*

Fig. 2

# Fig. 3

Fig.4

*Fig.5*

## Fig.6